# EUROPEAN PATENT SPECIFICATION

(11) **EP 4 452 908 B1**
(45) Date of publication and mention of the grant of the patent: **31.12.2025**
(21) Application number: 23773348.0
(22) Date of filing: 13.07.2023
(51) Int. Cl.: C06B 25/04, C07C 205/06, C07C 201/08

(54) **NITRATING MIXTURE FOR THE NITRATION OF 2,4- AND 2,6-DINITROTOLUENE TO 2,4,6-TRINITROTOLUENE AND A PROCESS FOR OBTAINING THEREOF USING THE SAME**
NITRIERUNGSGEMISCH ZUR NITRIERUNG VON 2,4- UND 2,6-DINITROTOLUOL ZU 2,4,6-TRINITROTOLUOL UND VERFAHREN ZU SEINER HERSTELLUNG UNTER VERWENDUNG DESSELBEN
MÉLANGE DE NITRATION POUR LA NITRATION DE 2,4-ET 2,6-DINITROTOLUÈNE EN 2,4,6-TRINITROTOLUÈNE ET SON PROCÉDÉ D'OBTENTION À L'AIDE DE CELUI-CI

(30) Priority: 13.07.2022 PL 44172622
(43) Date of publication of application: 30.10.2024
(73) Proprietor: POLITECHNIKA WARSZAWSKA, 00-661 Warszawa (PL)
(72) Inventor: MAKSIMOWSKI, Pawel, 05-822 Milanówek (PL); NASTALA, Andrzej, 37-418 Krzeszów (PL)
(74) Representative: Augustyniak, Magdalena Anna
(86) International application number: PCT/PL2023/050058
(87) International publication number: WO 2024/014973

(56) References cited:
- EP-B1- 2 841 412
- CN-C- 100 369 885
- RU-C2- 2 216 508
- US-A- 4 513 148

## Description

The invention relates to a nitrating mixture for the nitration of 2,4- and 2,6-dinitrotoluene to 2,4,6-trinitrotoluene and a process for obtaining thereof using the same.

2,4,6-trinitrotoluene (trotyl) is one of the most frequently synthesized explosives in the world. It is obtained as a result of the three-step nitration of toluene. In the last step, dinitrotoluene, both on a laboratory and industrial scale, is nitrated using a mixture consisting of fuming nitric acid (V), sulfuric acid (VI) and oleum (patents US2475095, US3742072). The use of this method is associated with the production of huge amounts of waste sulfuric acid (VI), the management of which is troublesome. Currently, it is recycled to the earlier stages of nitration after having been supplemented with nitric acid (V). After recycling to the first step and reacting, the acid is not suitable for use in the trotyl production process and is subjected to the regeneration process, which consists in: diluting with water to 70% and precipitating nitro compounds, denitrating (removal of nitric acid (V) residues and nitrogen oxides) and concentrating (removal of water). This process is associated with huge energy consumption and the formation of toxic nitrogen oxides. In addition, sulfuric acid (VI) poses a threat to the environment.

Nitration of toluene in the third step is also carried out at a high temperature (around 100°C) and is accompanied by side oxidation processes, during which large amounts of gases and tetranitromethane are released. For this reason, the operation of this process is dangerous and has been the cause of accidents in trotyl factories.

Other methods of nitration of dinitrotoluene to trinitrotoluene have been described in the literature. These are nitrations using the following nitrating mixtures: nitrogen oxide (V) in sulfuric acid (VI), nitric acid (V) in trifluoromethanesulfonic acid (US patent 7767868), nitric acid (V) and sulfuric acid (VI) in polyfluorohydrophenanthrene, nitric acid (V) and boron trifluoride (patent US3293 10), nitric acid (V) with tributylphosphine in supercritical carbon oxide (IV) (patent application US20070232840). Mixtures containing trifluoromethanesulfonic acid and boron trifluoride do not contain sulfuric acid (VI), but their components are harmful to the environment. The reaction of nitric acid (V) with tributylphosphine in supercritical carbon oxide (IV) requires high pressures, which would make the production of trotyl more expensive. All other mentioned systems contain sulfuric acid (VI).

A promising nitrating system seems to be nitro-oleum (a solution of nitrogen oxide (V) in nitric acid (V) ), however, the nitration reactions of dinitrotoluene to trinitrotoluene using this system are very slow and require the use of a large excess of the nitrating agent. CN100369885 discloses a method for preparing dinitrotoluene using a mixture comprising sulfuric acid, nitric acid and nitrogen oxide.

In the present invention, a nitrating mixture consisting of nitro-oleum and a smaller content of sulfuric acid (VI) than in the above-mentioned nitrating mixtures was used.

The invention relates to a nitrating mixture for the nitration of 2,4- and 2,6-dinitrotoluene to 2,4,6-trinitrotoluene, characterized in that it contains from 5 to 20 wt. % of nitrogen oxide (V), from 60 to 80 wt. % of nitric acid (V) and from 5 to 30 wt. % of sulfuric acid (VI).

Preferably, the nitrating mixture contains from 10 to 20 wt. % of nitrogen oxide (V), from 55 to 70 wt. % of nitric acid (V) and from 15 to 25 wt. % of sulfuric acid (VI).

The invention further relates to a process for obtaining of 2,4,6-trinitrotoluene comprising contacting 2,4- and 2,6-dinitrotoluene with the nitrating mixture according to the invention and heating the resulting reaction mixture, characterized in that the mass ratio of the nitrating mixture to 2,4- and 2,6-dinitrotoluene is not greater than 10:1.

Preferably, the process for obtaining of 2,4,6-trinitrotoluene according to the invention is characterized in that the mass ratio of the nitrating mixture to 2,4- and 2,6-dinitrotoluene is in the range from 5:1 to 8:1.

Preferably, the process for obtaining of 2,4,6-trinitrotoluene according to the invention is characterized in that the nitration reaction is carried out at a temperature of 50 to 80°C. More preferably, the nitration reaction is carried out at a temperature of 60 to 70°C.

Preferably, the process for obtaining of 2,4,6-trinitrotoluene according to the invention is characterized in that the nitration reaction is carried out for not less than 4 hours.

Table 1 compares the amount of waste sulfuric acid (VI) for different processes for obtaining of TNT.

**Table 1. Consumption of sulfuric acid (VI) per 1 t of TNT for various methods for obtaining of trotyl**

| Method of nitration of DNT to TNT | Amount of sulfuric acid (VI) per 1 t of TNT [kg] | Reaction temperature [°C ] | Yield [%] |
|---|---|---|---|
| Traditional | 2240 | 100 | 87 |
| Nitric oxide (V) in sulfuric acid (VI) | 8704 | 77 | 93 |
| According to the invention | 1154 | 70 | 84 |

Based on Table 1, it can be noticed that the method allows for much lower consumption of sulfuric acid (VI) in trotyl-producing plants. The decrease amounts to 38% compared to the traditional method. It may be of particular importance when nitrating agents without sulfuric acid (VI) are used in the first and second stages of nitration, e.g. fuming nitric acid (V) or nitrogen oxide (V) in an organic solvent.

Moreover, carrying out the nitration according to the present invention demonstrates a lower level of secondary oxidation processes and a lower process temperature than the traditional method, which increases safety of the process. In this method, it is possible to recycle nitric acid (V) and reuse it to prepare a solution of nitrogen oxide (V) in nitric acid (V).

### Working examples

### Example 1

52 g of 2,4-dinitrotoluene, 200 g of 20% nitro-oleum and 60 g of 99% sulfuric acid (VI) were added to a three-necked, round-bottom flask equipped with a thermometer and an ball reflux condenser (Allihna), located on a magnetic stirrer, in a water bath. After adding the ingredients, stirring was started and the contents of the flask were heated to 70°C. From the moment of reaching the set temperature, the reaction was carried out for 8 hours. After the set time had elapsed, the content of the flask were cooled to room temperature and poured into a beaker with water and ice. The precipitate was filtered off on a Schott G2 funnel and washed with water until pH=7. The precipitate was transferred to a crystallizer and dried in a drier to a constant weight at 60°C. The yield of the reaction was 85%, and the trotyl content in the sample determined by GC-MS was 99.85%.

### Example 2

10 g of 2,4-dinitrotoluene and a nitrating mixture indicated in Table 2, containing 20 % of nitro-oleum and min. 99 % pure sulfuric acid (VI), were added to a 100 mL three-neck, round-bottom flask, equipped with a magnetic stirrer, thermometer, and ball reflux condenser (Allihna), and located in a water bath. The reaction was carried out for 8 hours. Upon completion of the reaction, the contents of the flask were poured into a beaker of water and ice and then extracted with dichloromethane (1 x 50 mL). The extract was washed twice with 0.5% sodium bicarbonate solution and then with distilled water to pH=7. Then, a small amount of magnesium sulphate (VI) was poured into the extract and left overnight, and then the sample was filtered through a fluted filter and the solvent was distilled off on a vacuum evaporator. The results of nitrations and sample compositions of the nitrating mixtures are presented in Table 2.

**Table 2. Effect of the nitrating mixture composition on the degree of conversion of DNT to TNT**

| Amount of DNT [g] | Amount of the nitrating mixture [g] | Nitrating mixture composition | | | |
|---|---|---|---|---|---|
| | | N₂O₅ [%] | HNO₃ [Y^{o}] | H₂SO₄ [%] | Yield [%] |
| 10 | 50 | 17.8 | 78.0 | 4.2 | 36 |
| 10 | 50 | 15.5 | 75.8 | 8.7 | 59 |
| 10 | 50 | 10.9 | 71.8 | 17.3 | 68 |
| 10 | 50 | 9.6 | 70.6 | 19.8 | 79 |
| 10 | 50 | 7.9 | 69.1 | 23.0 | 85 |
| 10 | 50 | 7.5 | 68.8 | 23.7 | 86 |
| 10 | 50 | 5.6 | 67.0 | 27.4 | 91 |

### Example 3

The reaction was carried out analogously to example 1 except of changing the amount of 99% sulfuric acid (VI) used in the nitrating mixture per 1 g of 2,4-DNT, the nitration reaction time and the temperature. The results are presented in table 3.

**Table 3. Effect of reaction parameters deviation on the degree of conversion and reaction yield**

| Temperature [°C] | Grams of 99% sulfuric acid/1 g of DNT | Reaction time [h] | TNT yield [%] |
|---|---|---|---|
| 70 | 1.37 | 4 | 74 |
| 70 | 1.37 | 6 | 78 |
| 70 | 1.37 | 8 | 85 |
| 60 | 1.37 | 6 | 70 |
| 80 | 1.37 | 6 | 75 |
| 70 | 0.89 | 6 | 63 |
| 70 | 1.78 | 6 | 82 |

The composition of the nitrating mixture according to the invention and the disclosed process can be used for the production of the explosive 2,4,6- trinitrotoluene (trotyl).

## Claims

1. A nitrating mixture for the nitration of 2,4- and 2,6-dinitrotoluene to 2,4,6-trinitrotoluene, **characterized in that** it contains from 5 to 20 wt. % of nitrogen oxide (V), from 60 to 80 wt. % of nitric acid (V) and from 5 to 30 wt. % of sulfuric acid (VI).

2. The process for obtaining of 2,4,6-trinitrotoluene comprising contacting 2,4- and 2,6-dinitrotoluene with the nitrating mixture as defined in claim 1, and heating the reaction mixture thus formed, **characterized in that** the mass ratio of the nitrating mixture to 2,4- and 2,6-dinitrotoluene is not greater than 10:1.

3. The process for obtaining of 2,4,6-trinitrotoluene according to claim 2, **characterized in that** the mass ratio of the nitrating mixture to 2,4- and 2,6-dinitrotoluene is in the range of 5:1 to 8:1.

4. The process for obtaining of 2,4,6-trinitrotoluene according to claim 2 or 3, **characterized in that** the nitration reaction is carried out at a temperature of 50 to 80°C.

5. The process for obtaining of 2,4,6-trinitrotoluene according to claim 4, **characterized in that** the nitration reaction is carried out at a temperature of 60 to 70°C.

6. The process for obtaining of 2,4,6-trinitrotoluene according to any one of claims 2 to 5, **characterized in that** the nitration reaction is carried out for not less than 4 hours.

## Patentansprüche

1. Ein Nitriergemisch zur Nitrierung von 2,4-Dinitrotoluol und 2,6-Dinitrotoluol zu 2,4,6-Trinitrotoluol, **dadurch gekennzeichnet, dass** es 5 bis 20 Gew.-% Stickstoffoxid (V), 60 bis 80 Gew.-% Salpetersäure (V) und 5 bis 30 Gew.-% Schwefelsäure (VI) enthält.

2. Verfahren zur Gewinnung von 2,4,6-Trinitrotoluol durch Inkontaktbringen von 2,4-Dinitrotoluol und 2,6-Dinitrotoluol mit dem Nitriergemisch gemäß Anspruch 1 und Erhitzen des so gebildeten Reaktionsgemisches, **dadurch gekennzeichnet, dass** das Massenverhältnis des Nitriergemisches zu 2,4-Dinitrotoluol und 2,6-Dinitrotoluol nicht größer als 10:1 ist.

3. Verfahren zur Gewinnung von 2,4,6-Trinitrotoluol gemäß Anspruch 2, **dadurch gekennzeichnet, dass** das Massenverhältnis des Nitriergemisches zu 2,4-Dinitrotoluol und 2,6-Dinitrotoluol im Bereich von 5:1 bis 8:1 liegt.

4. Verfahren zur Gewinnung von 2,4,6-Trinitrotoluol nach Anspruch 2 oder 3, **dadurch gekennzeichnet, dass** die Nitrierungsreaktion bei einer Temperatur von 50 bis 80 °C durchgeführt wird.

5. Verfahren zur Gewinnung von 2,4,6-Trinitrotoluol nach Anspruch 4, **dadurch gekennzeichnet, dass** die Nitrierungsreaktion bei einer Temperatur von 60 bis 70 °C durchgeführt wird.

6. Verfahren zur Gewinnung von 2,4,6-Trinitrotoluol nach einem der Ansprüche 2 bis 5, **dadurch gekennzeichnet, dass** die Nitrierungsreaktion mindestens 4 Stunden lang durchgeführt wird.

## Revendications

1. Mélange nitrant pour la nitration du 2,4-dinitrotoluène et 2,6-dinitrotoluène en 2,4,6-trinitrotoluène, **caractérisé en ce qu'il** contient de 5 à 20 % en poids d'oxyde d'azote (V), de 60 à 80 % en poids d'acide nitrique (V) et de 5 à 30 % en poids d'acide sulfurique (VI).

2. Procédé d'obtention de 2,4,6-trinitrotoluène comprenant la mise en contact de 2,4-dinitrotoluène et 2,6-dinitrotoluène avec le mélange nitrant tel que défini dans la revendication 1, puis le chauffage du mélange réactionnel ainsi formé, **caractérisé en ce que** le rapport massique du mélange nitrant au 2,4-dinitrotoluène et 2,6-dinitrotoluène n'est pas supérieur à 10:1.

3. Procédé d'obtention de 2,4,6-trinitrotoluène selon la revendication 2, **caractérisé en ce que** le rapport massique du mélange nitrant au 2,4-dinitrotoluène et 2,6-dinitrotoluène est compris entre 5:1 et 8: 1.

4. Procédé d'obtention de 2,4,6-trinitrotoluène selon la revendication 2 ou 3, **caractérisé en ce que** la réaction de nitration est réalisée à une température comprise entre 50 et 80 °C.

5. Procédé d'obtention de 2,4,6-trinitrotoluène selon la revendication 4, **caractérisé en ce que** la réaction de nitration est réalisée à une température comprise entre 60 et 70 °C.

6. Procédé d'obtention de 2,4,6-trinitrotoluène selon l'une quelconque des revendications 2 à 5, **caractérisé en ce que** la réaction de nitration est réalisée pendant au moins 4 heures.
